# EUROPEAN PATENT APPLICATION

(11) **EP 2 369 339 A1**
(43) Date of publication of application: **28.09.2011**
(21) Application number: 11158561.8
(22) Date of filing: 16.03.2011
(51) Int. Cl.: G01N 33/38

(54) **Electronic device for monitoring the physical characteristics of concrete castings**

(30) Priority: 24.03.2010 IT MI20100488
(71) Applicant: Tecnosoft s.r.l., 20068 Peschiera Borromeo (MI) (IT); CROSS POINT S.R.L., 20035 Lissone, Monza e Brianza (IT)
(72) Inventor: Rossi, Sergio Libero Cassiano, 20068, PESCHIERA BORROMEO MI (IT)
(74) Representative: Modiano, Micaela Nadia

(57) **Abstract**

An electronic device (1) for monitoring the physical characteristics of concrete castings, comprising electronic data processing and management means (4), which are functionally connected to means (5) for detecting physical characteristics of concrete castings and to data transfer means (13), which can be connected to at least one remote monitoring station (14) for the transfer of the data measured by the detection means (5) to the remote monitoring station (14) and for programming the electronic data processing and management means (4), the electronic data processing and management means (4), the detection means (5) and the data transfer means (13) being embeddable in a concrete casting (3) to monitor the physical characteristics of the casting (3).

## Description

The present invention relates to an electronic device for monitoring the physical characteristics of concrete castings, which is adapted to identify, even years later, fraud in the composition of the concrete.

The strength of concrete castings is currently verified by testing specimens obtained from the concrete of the casting.

In order to pass the strength tests, although a low-quality concrete has been used, dishonest builders may use one type of concrete for the specimens and another type for the casting.

In order to identify this fraud, it is known in the art to test a core taken from the casting.

More specifically, the strength of the casting, which increases as the concrete cures, i.e., as time goes by, is verified on the specimens by performing compression tests at successive times after casting and according to strict standards.

Subsequently, more specifically when the concrete has cured sufficiently, it is possible to proceed with the construction of further structures that must be supported by the components built by means of the casting only when the tests on the specimens indicate a sufficient strength.

In particular, some physical parameters can be correlated with the curing of the concrete. The most important ones are the trend of the temperature over time and the variation of the electrical conductivity over time.

As regards the measurement of the temperature variation over time, which depends on the quantity of water that is present in the cement, on the quality of the cement, on the quantity of sand and inert material, during curing the cement generates heat, known as hydration heat, according to well-established curves and as a function of the type of cement, so as to able to make predictions regarding the curing of the concrete.

Quite often, strength values which would allow to continue construction work, with consequent saving of construction time, are reached between one test and the next.

This prediction can be made by measuring the temperature of the concrete casting with disposable temperature probes according to a method which is regulated for example by the ASTM C1074 standards.

In this manner, by analyzing the trend of the temperature of the sample over time a direct verification of the curing of the concrete of the casting can be obtained, since two identical castings have substantially identical temperature trends over time, whereas different castings have different trends.

As regards the variation in electrical conductivity over time, electrical conductivity tests are known which are performed in order to correlate the strength of specimens with their electrical conductivity.

More specifically, since electrical conduction in solutions occurs by ion displacement and not due to the flow of electrons, the progressive hardening of the cement hinders ion displacement and provides, by examining the electrical conductivity data, information on the curing process and on its duration.

In practice, the temperature and electrical conductivity measurements are obtained by embedding thermometers and electrodes in the component and in the reference samples, their electronic portion being external so that it can be applied alternately to the sensors embedded in the reference samples and in the component and so that it can be reused several times for further tests on other castings and specimens.

The correlations between the temperature and electrical conductivity measurements performed both on the samples and on the component during the curing of the concrete allow to verify whether the specimens and the component have the same characteristics and therefore are meant to prevent any fraud on the part of builders.

For example, one of the most common types of fraud is the use of salty sea sand instead of river sand. This use is forbidden, since sea sand causes corrosion of the rods of the reinforced concrete.

A mix with sea sand in fact has a different electrical conductivity with respect to a mix with river sand, and the measurement of the conductivity of a mix with sea sand differs from that obtained from a mix with river sand, allowing to detect the fraud.

Likewise, the measurement of the trend of the temperature during the curing of the concrete, if compared with standard data related to identical compositions, helps to detect any fraud.

These measurement methods of a known type are not devoid of drawbacks, which include the fact that as construction work proceeds, it is no longer possible to take measurements to monitor the concrete with which the component is made in order to check the match between the concrete with which the component is actually made and the concrete of the specimens obtained theoretically from it or of standard mixes.

The aim of the present invention is to provide an electronic device for monitoring the physical characteristics of concrete castings which is adapted to detect, even years later, fraud in the composition of the concrete.

Within this aim, an object of the present invention is to provide an electronic device that is simple to provide, simple to use, and has a low cost.

This aim and these and other objects that will become better apparent hereinafter, are achieved by an electronic device for monitoring the physical characteristics of concrete castings, characterized in that it comprises electronic data processing and management means, which are functionally connected to means for detecting physical characteristics of concrete castings and to data transfer means, which can be connected to at least one remote monitoring station for the transfer of the data measured by said detection means to said at least one remote monitoring station and for programming said electronic data processing and management means, said electronic data processing and management means, said detection means and said data transfer means being embeddable in a concrete casting to monitor the physical characteristics of said casting.

Further characteristics and advantages of the present invention will become apparent from the description of a preferred but not exclusive embodiment of an electronic device for monitoring the physical characteristics of concrete castings, according to the invention, illustrated by way of non-limiting example in the accompanying drawings, wherein:

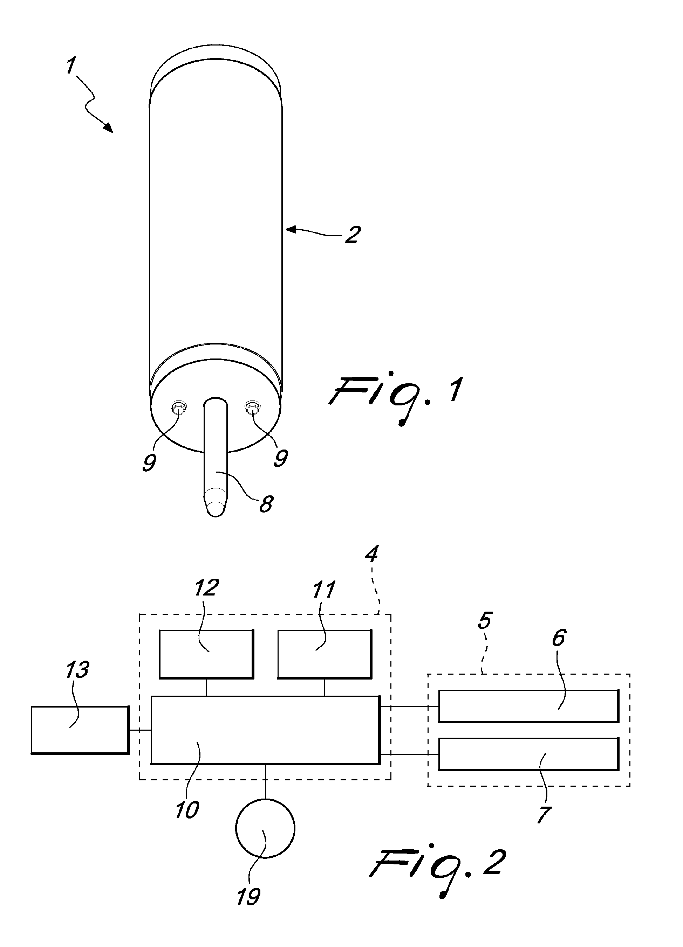

Figure 1 is a side elevation view of a preferred but not exclusive embodiment of an electronic device for monitoring the physical characteristics of concrete castings, according to the invention;
Figure 2 is an electrical schematic of the electronic device shown in Figure 1;

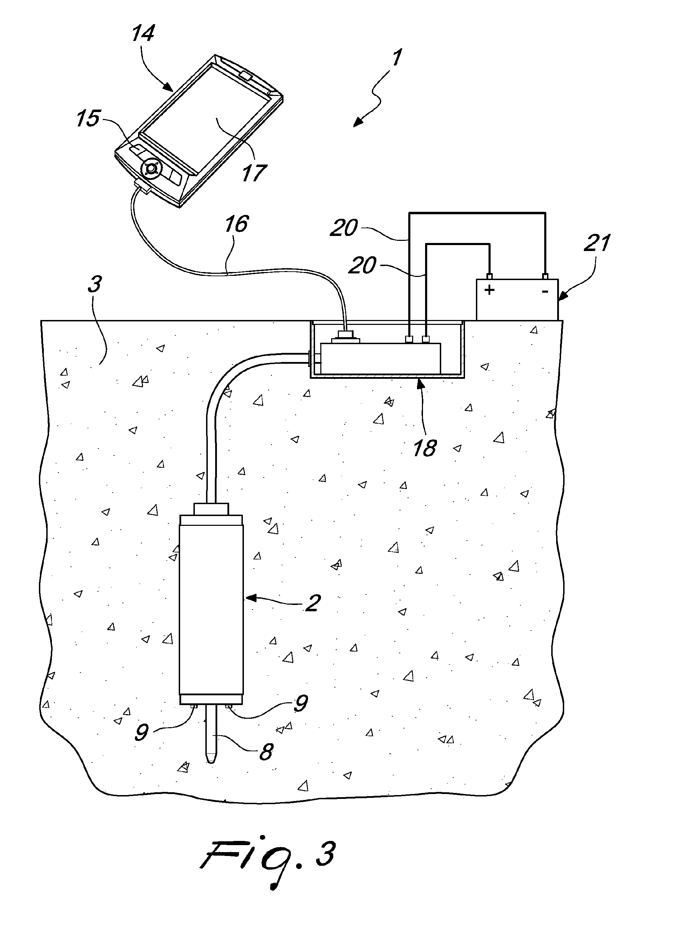

Figure 3 is a side elevation view of the electronic device shown in Figure 1 during use;

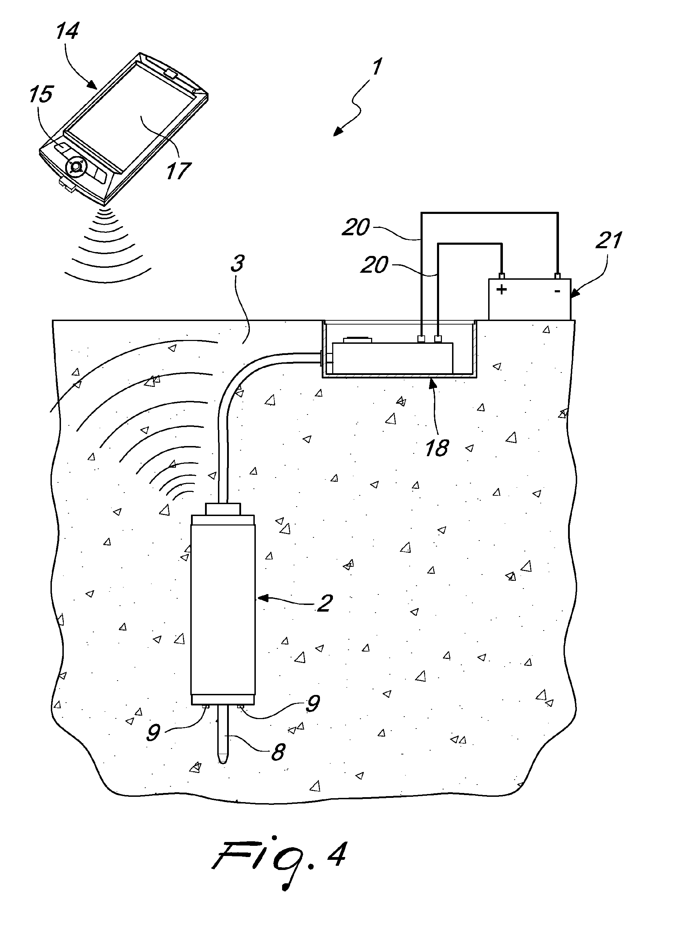

Figure 4 is a side elevation view of a variation of the electronic device shown in Figure 1 during use.

With reference to the figures, the electronic device for monitoring the physical characteristics of concrete castings, generally designated by the reference numeral 1, comprises a hermetic box-like body 2, which can be embedded in a concrete casting 3 and accommodates internally electronic data processing and management means 4, which are functionally connected to means 5 for detecting physical characteristics of concrete castings which are partly accommodated in the box-like body 2 and are partly external thereto.

More precisely, the detection means 5, which comprise a temperature measurement circuit 6 and/or an electrical conductivity measurement circuit 7 or both, as shown in the figures, have sensing elements 8 and 9 arranged outside the box-like body 2.

In particular, a temperature sensor 8 and two electrodes 9 for measuring electrical conductivity are provided.

For example, the box-like body 2 can have a substantially cylindrical shape with a circular base which has a diameter on the order of millimeters and a length on the order of a few tens of millimeters.

Advantageously, the electronic data processing and management means 4 comprise a microprocessor 10 and at least a memory 11 of the non-volatile type, such as for example a FLASH, EPROM, FRAM memory or the like, in order to store the measurements made by the detection means 5.

Moreover, as will be described further hereinafter, the electronic data processing and management means 4 comprise timer means 12 for interrupting the acquisition of data by the detection means 5 after a preset time.

Data transfer means 13 are further comprised within the box-like body 2 and are functionally connected to the electronic data processing and management means 4 and can be connected to at least one remote monitoring station 14 for the transfer of the data measured from the detection means 5 to said remote monitoring station 14 and for the programming of said electronic data processing and management means 4 by means of a user interface 15 with which the remote monitoring station 4 is provided.

More specifically, the data transfer means 13 comprise at least two wires 16, which end in a junction box 18, which is permanently embedded in the structure and is rendered accessible for the temporary connection of the electronic device 1 to the remote monitoring station 14, which can consist, for example, of a handheld device or a portable computer provided with a graphical interface 17.

Alternately or in parallel, as will be described in greater detail hereinafter, the connection between the electronic device 1 and the remote monitoring station 14 can occur by means of radio transceivers associated with the electronic data processing and management means 4 and the remote monitoring station 14.

The entire electronic device 1 is powered by power supply means 19, which are also accommodated within the box-like body 2 and consist of at least one long-life internal battery, for example of the lithium type, with a life substantially equal to 10 years, which is connected directly to the electronic data processing and management means 4.

If said battery is exhausted, by means of power supply cables 20, also reachable by means of the junction box 18, it is possible to connect the electronic device 1 to an external power source 21.

In this manner, all the electronic components of the electronic device 1, i.e., the electronic data processing and management means 4, the detection means 5 and the data transfer means 13, can be embedded in the concrete casting 3 to monitor the physical characteristics of the casting 3, both if it is the component and if it is the specimen.

The application of the electronic device 1 for monitoring the physical characteristics of concrete castings is described hereinafter.

In order to perform the checks required to establish that the concrete of the component corresponds to the concrete of which the specimen is made, i.e., in order to establish the occurrence of fraud, an electronic device 1 is embedded in the component and another one in the specimen obtained in theory from the same mix as the casting with which the component is produced.

By means of the user interface 15 and 18 with which the remote monitoring station 14 is equipped, it is possible to program the start of the acquisition and subsequently the reading of the data acquired by the electronic device 1 and stored in its memory 11.

The long-life internal battery of the power supply means 19 allows operation in acquisition for the required time, generally one month, and keeps active the possibility to communicate and download the acquired data for at least 10 years.

As already mentioned, communication occurs by means of two wires 16, reference and input/output, which are carried into a junction box 18 which is embedded permanently in the structure and is rendered accessible for future readings of the device or by radio transceiving.

The electronic device 1 can be read for a longer time if the power supply cables 20 are also carried outside in order to connect it to the external power source 21 to replace the internal battery, which may have drained after 10 years.

Among the functionalities and characteristics of the electronic device 1, it is noted that acquisition cannot be stopped, the data can have a format that cannot be modified, the electronic devices 1 themselves may have a unique and non-modifiable serial number so as to discourage and/or prevent any tampering.

More specifically, acquisition is of the dynamic type and storage can occur for variations of the measurement of programmed quantities: for example, if the programmed variation is 0.1 °C, storage occurs every 0.1 °C and the record shall relate to the date, time and temperature. In this manner there will be more records at the beginning of the process, where the variations of the measured values are greater.

The files related to the measurements of the component and of the specimen can be transferred via the Internet to the control authorities; control can be automatic.

Moreover, the specimen containing the reference electronic device 1, according to the invention, must be subjected to a strength test at the end of the curing cycle, in order to document that the same poor mix was not used both in the component and in the specimen. The strength of the specimen with the electronic device 1 embedded therein must be similar to the strength of the standard specimen.

Moreover, the electronic device 1 is capable of detecting, but not of distinguishing the nature, by comparison with the specimen, of different qualities of cement and/or dilution of the mix and/or different inert material and presence of sea sand.

Comparison of the data obtained from the component and from the specimen and optionally with the predefined standard values is documentation of the correct process.

In practice it has been found that the electronic device for monitoring the physical characteristics of concrete castings, according to the present invention, fully achieves the intended aim and objects, since it allows to perform checks of the curing of the concrete in an installed component at any time during its production, without halting the work linked thereto, and to perform checks regarding the match of the concrete of which the component is actually made and the concrete of which the specimen associated therewith is made.

If the components of the casting and the electrical conductivity of the fresh water used are known, an automatic comparison via telematic means of all castings is in fact possible if a data bank with castings of the same type is generated: it is sufficient to transfer the files over the Internet to the data bank to point out anomalies and perform the necessary controls.

This prevents any connivance among builders, project managers and testing laboratories, since it is always possible to verify the truthfulness of the data by reading the electronic device according to the invention, embedded in the component, even years later.

Another advantage of the electronic device according to the present invention is that by means of a comparison of the curves recorded by the electronic devices of the component and of the specimen, and optionally with the data of standard mixes, it is possible to report any anomalies.

A further advantage of the electronic device according to the present invention is that it is economically competitive with respect to the background art.

The electronic device for monitoring the physical characteristics of concrete castings thus conceived is susceptible of numerous modifications and variations, all of which are within the scope of the appended claims.

All the details may further be replaced with other technically equivalent elements.

In practice, the materials used, so long as they are compatible with the specific use, as well as the contingent shapes and dimensions, may be any according to requirements and to the state of the art.

The disclosures in Italian Patent Application No. MI2010A000488 from which this application claims priority are incorporated herein by reference.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. An electronic device (1) for monitoring the physical characteristics of concrete castings, **characterized in that** it comprises electronic data processing and management means (4), which are functionally connected to means (5) for detecting physical characteristics of concrete castings and to data transfer means (13), which can be connected to at least one remote monitoring station (14) for the transfer of the data measured by said detection means (5) to said at least one remote monitoring station (14) and for programming said electronic data processing and management means (4), said electronic data processing and management means (4), said detection means (5) and said data transfer means (13) being embeddable in a concrete casting (3) to monitor the physical characteristics of said casting (3).

2. The electronic device (1) according to claim 1, **characterized in that** said detection means (5) comprise at least a temperature measurement circuit (6) and/or a circuit (7) for measuring electrical conductivity

3. The electronic device (1) according to one or more of the preceding claims, **characterized in that** said electronic data processing and management means (4) comprise at least one microprocessor (10) and at least one memory (11).

4. The electronic device (1) according to one or more of the preceding claims, **characterized in that** said memory (11) is of the non-volatile type.

5. The electronic device (1) according to one or more of the preceding claims, **characterized in that** said electronic data processing and management means (4) comprise timer means (12) for interrupting the acquisition of data on the part of said detection means (5) after a preset time.

6. The electronic device (1) according to one or more of the preceding claims, **characterized in that** it comprises means (19) for supplying power to said electronic data processing and management means (4).

7. The electronic device (1) according to one or more of the preceding claims, **characterized in that** said power supply means (19) comprise at least one long-life battery.

8. The electronic device (1) according to one or more of the preceding claims, **characterized in that** said power supply means (19) comprise power supply cables (20), which can be connected to an external power source (21).

9. The electronic device (1) according to one or more of the preceding claims, **characterized in that** it comprises a hermetic box-like body (2), which can be embedded in said concrete casting (3) and accommodates said electronic data processing and management means (4), said data transfer means (13), said power supply means (19) and said detection means (5) being accommodated partly in said box-like body (2) and partly in a junction box (18), which can be accessed for the temporary connection of said electronic device (1) to said remote monitoring station (14) and having the sensing elements (8, 9) arranged outside said box-like body (2).

10. The electronic device (1) according to one or more of the preceding claims, **characterized in that** said data transfer means (13) comprise transceivers which are associated with said electronic data processing and management means (4) and with said remote monitoring station (14).
